# EUROPÄISCHE PATENTSCHRIFT

(11) **EP 2 823 801 B1**
(45) Veröffentlichungstag und Bekanntmachung des Hinweises auf die Patenterteilung: **19.04.2017**
(21) Anmeldenummer: 13176333.6
(22) Anmeldetag: 12.07.2013
(51) Int. Cl.: A61K 6/00, A61K 6/083

(54) **Dentalmaterialien auf der Basis von harnstoffgruppenhaltigen Monomeren**
Dental materials on the basis of monomers containing urea groups
Matériau dentaire à base de monomères contenant des groupes urée

(43) Veröffentlichungstag der Anmeldung: 14.01.2015
(73) Patentinhaber: Ivoclar Vivadent AG, 9494 Schaan (LI)
(72) Erfinder: Moszner, Norbert, 9493 Mauren (LI); Catel, Yohann, 9470 Buchs (CH)
(74) Vertreter: Muth, Heinz-Peter

(56) Entgegenhaltungen:
- US-A- 3 488 330
- US-A- 5 292 619
- US-A1- 2011 281 025
- US-A1- 2013 109 780
- US-B1- 6 943 211

## Beschreibung

Die vorliegende Erfindung betrifft Dentalmaterialien auf der Basis von harnstoffgruppenhaltigen Monomeren. Die Dentalwerkstoffe eigenen sich besonders als Zahnfüllungsmaterialien (Komposite), Adhäsive, Zemente oder Beschichtungsmaterialien.

Die polymerisierbare organische Matrix von dentalen Adhäsiven, Zementen oder Kompositen besteht vor allem aus einer Mischung von Monomeren, Initiator-Komponenten, Stabilisatoren und Pigmenten. Als Harze werden meistens Mischungen von Dimethacrylaten eingesetzt (N. Moszner, T. Hirt, J. Polym. Sci. Part A: Polym. Chem. 50 (2012) 4369-4402).

Bei den Matrixmonomeren unterscheidet man zwischen nichtaciden und aciden Monomethacrylaten, vernetzenden Dimethacrylaten und multifunktionellen Methacrylaten. Radikalisch polymerisierbare Monomere mit aciden Gruppen verleihen Dentalmaterialien selbstätzende Eigenschaften, so dass auf eine Säurebehandlung der Zahnoberfläche vor dem Auftrag der Materialien zum Anätzen der Zahnoberfläche und zum Entfernen der sogenannten Schmierschicht verzichtet werden kann. Außerdem verbessern sie durch ionische oder kovalente Wechselwirkungen mit der Zahnsubstanz die Haftung am Zahn. Sie werden primär in Adhäsiven und selbstätzenden Dentalwerkstoffen eingesetzt (N. Moszner, U. Salz, J. Zimmermann, Dent. Mat. 21 (2005) 895-910).

Die Anbindung der radikalisch polymerisierbaren Gruppe (n) an das Molekül erfolgt bei bekannten Dentalmonomeren hauptsächlich über Ester-, Ether- und Urethangruppen. Es sind aber auch Monomere bekannt, bei denen die Anbindung über andere Gruppen wie beispielsweise Harnstoffgruppen erfolgt.

Aus der EP 0 209 700 A2 sind Dentalmaterialien auf der Basis von polymerisierbaren Tricyclodecanderivaten bekannt. Die Tricyclodecanderivate weisen zwei (Meth)acrylsäurgruppen auf, die über Urethan- oder Harnstoffgruppen an den Tricyclodecanrest gebunden sind.

EP 0 400 383 A1 offenbart Harnstoffgruppen enthaltende (Meth)acrylsäurederivate von Triisocyanaten. Diese sollen sich zur Verwendung in Dentalmaterialien eignen. Die (Meth)acrylsäurederivate haben einen bicyclischen Grundkörper wie Diphenylmethan oder Dicyclohexylmethan.

Die WO 2009/006282 A2 betrifft Dentalmaterialien auf der Basis von polyfunktionellen (Meth)acrylaten, die Harnstoff-, Amid- oder vorzugsweise Urethangruppen enthalten.

Die WO 2008/033911 A2 offenbart radikalisch polymerisierbare Monomere, die Harnstoffgruppen enthalten können und die als Gelbildner in dentalen Zusammensetzungen eingesetzt werden. Unter Gelbildnern werden niedermolekulare Stoffe verstanden, die ein dreidimensionales Netzwerk bilden, wenn sie in einer organischen Flüssigkeit gelöst werden, und die dabei die Flüssigkeit unter Ausbildung eines nicht fließfähiges Gels immobilisieren. Das Gel lässt sich durch Temperaturerhöhung reversibel verflüssigen.

Von den genannten Schriften beschreibt allein die WO 2008/033911 A2 konkrete Dentalmaterialien, die harnstoffgruppenhaltige Monomere enthalten. Diese Monomere bilden durch Selbstorganisation physikalische Netzwerke, welche die Zusammensetzung verfestigen und ein Gel mit wachsartigen Eigenschaften ergeben. Die Gelbildung ist für viele Anwendungsformen nachteilig, weil sie mit einer drastischen Viskositätserhöhung verbunden ist, welche z.B. die Anwendung von Kompositen und Adhäsiven erheblich erschwert. Die Gelbildung wirkt sich außerdem nachteilig auf die Polymerisationsgeschwindigkeit aus.

Dentalmaterialien müssen eine Vielzahl unterschiedlicher Anforderungen erfüllen. Sie müssen eine gute Langzeitstabilität gegenüber der vorzeitigen Härtung bei der Lagerung aufweisen, andererseits aber auch eine hohe Polymerisationsrate bei der radikalischen Homo- und Copolymerisation zeigen. Sie sollen unter oralen Bedingungen stabil und lichtbeständig sein und nicht zu Verfärbungen neigen. Außerdem sollen sie eine geringe orale Toxizität und bei der Polymerisation nur einen geringen Schrumpf aufweisen. Darüber hinaus soll die Wassersorption nach der Härtung klein sein, insbesondere bei Adhäsiven ist aber vor der Härtung eine gute Mischbarkeit mit polaren Lösungsmitteln und deren Mischungen mit Wasser vorteilhaft.

Bekannte Dentalmaterialien enthalten Monomere, die hinsichtlich bestimmter Eigenschaften optimiert wurden. Monomere, die alle gestellten Anforderungen in gleicher Weise erfüllen, sind nicht bekannt, so dass nach wie vor Bedarf an verbesserten Matrixmonomeren besteht. Harnstoffgruppen enthaltende Monomere haben bisher keine praktische Anwendung gefunden.

Der Erfindung liegt die Aufgabe zugrunde, Dentalwerkstoffe bereitzustellen, welche die genannten Anforderungen erfüllen und ein für dentale Zwecke optimales Eigenschaftsprofil haben und insbesondere eine hohe Polymerisationsrate bei der radikalischen Polymerisation aufweisen. Außerdem sollen die Dentalwerkstoffe nach der Polymerisation über gute mechanische Eigenschaften verfügen. Zudem sollen sie eine hohe Haftung an der Zahnhartsubstanz (Dentin und insbesondere Schmelz) aufweisen, und sich besonders als Adhäsive, Zemente, Komposite oder Beschichtungsmaterialien eignen. Darüber hinaus ist auch eine gute Löslichkeit in polaren Lösungsmitteln und in Mischungen aus polaren Lösungsmitteln und Wasser wünschenswert.

Die Aufgabe wird erfindungsgemäß durch Dentalwerkstoffe gelöst, die mindestens ein Harnstoffderivat gemäß der allgemeinen Formel I enthalten, 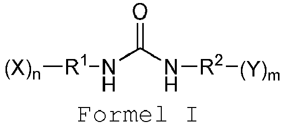 in der
- R¹, R² =: unabhängig voneinander jeweils ein aliphatischer C₁-C₁₅-Rest sind, der durch -O-, -S-, -CO-O-, unterbrochen sein kann,
- X =: eine radikalisch polymerisierbare Gruppe ist,
- Y =: eine radikalisch polymerisierbare Gruppe oder eine acide Gruppe ist,
- n, m =: unabhängig voneinander jeweils 1, 2, oder 3 sind.

Bevorzugte radikalisch polymerisierbare Gruppen sind CH₂=CR³-CO-Z- oder R⁴O-CO-C(=CH₂)-CH₂-Z-, wobei Z O oder NR⁵ ist oder entfällt, R³ H oder CH₃ ist und R⁴ und R⁵ unabhängig voneinander jeweils H oder C₁-C₇-Alkyl sind. Besonders bevorzugt sind (Meth)acryloyloxygruppen (CH₂=CR³-CO-Z- mit Z = O), insbesondere (Meth)acryloylaminogruppen (CH₂=CR³-CO-Z- mit Z = NR⁵) und R⁴O-CO-C(=CH₂)-CH₂-Z-, wobei Z vorzugsweise O ist; R³ ist jeweils H oder CH₃ und R⁴ und R⁵ sind unabhängig voneinander jeweils CH₃ oder C₂H₅.

Bevorzugte acide Gruppen sind oder -PO(OH)2, -O-PO(OH)₂ und -SO₃H.

Gemäß einer besonders bevorzugten Ausführungsform haben X und Y die folgenden Bedeutungen:
- X =: CH₂=CR³-CO-Z- oder R⁴O-CO-C(=CH₂)-CH₂-Z-, wobei Z O oder NR⁵ ist oder entfällt, R³ H oder CH₃ ist und R⁴ und R⁵ unabhängig voneinander jeweils H oder C₁-C₇-Alkyl sind, und
- Y =: entweder CH₂=CR^{3'}-CO-Z'- oder R^{4'}O-CO-C(=CH₂)-CH₂-Z'-, wobei Z' O oder NR^{5'} ist oder entfällt, R^{3'} H oder CH₃ ist und R^{4'} und R^{5'} unabhängig voneinander jeweils H oder C₁-C₇-Alkyl sind; oder -PO(OH)₂, -O-PO(OH)₂, -SO₃H.

Die Gruppen R¹ und R² sind aliphatischen Gruppe, die n-fach durch den Rest X bzw. m-fach durch Y substituiert sind. Diese Gruppen können verzweigt oder geradkettig sein. Die Formel I erfasst nur solche Verbindungen, die mit der chemischen Valenzlehre vereinbar sind.

Vorzugsweise sind R¹ und R² lineare aliphatische Reste, insbesondere Reste mit der Formel (CH₂)ₚ wobei p eine ganze Zahl von 1 bis 15, besonders bevorzugt 2 bis 12 und ganz besonders bevorzugt 3 bis 10 ist. R¹ und R² können unterschiedlich oder vorzugsweise gleich sein. Lineare Reste sind besonders jeweils dann bevorzugt, wenn n bzw. m 1 sind. Wenn n bzw. m größer als 1 sind, haben R¹ bzw. R² vorzugsweise eine verzweigte Struktur. In diesem Fall sind die mehreren Gruppen X bzw. Y vorzugsweise auf die Haupt- und die Seitenkette(n) verteilt.

Der Hinweis, dass ein Rest durch Heteroatome oder funktionelle Gruppen unterbrochen ist, ist so zu verstehen, dass die Heteroatome oder funktionellen Gruppen in die Kohlenstoffkette eingeschoben werden und beidseitig von C-Atomen begrenzt sind. Die Aneinanderreihung von Heterotatomen und/oder funktionellen Gruppen fällt nicht unter diese Definition.

Vorzugsweise ist R¹ nicht oder durch 1 bis 4, insbesondere 1 bis 2 Heteroatome oder funktionelle Gruppen unterbrochen, besonders bevorzugt durch 1 oder 2 O-Atome. R² ist vorzugsweise nicht oder durch 1 bis 4, insbesondere 1 bis 2 Heteroatome oder funktionelle Gruppen unterbrochen, besonders bevorzugt durch 1 oder 2 O-Atome.

Die hierin genannten bevorzugten Definitionen der Variablen können unabhängig voneinander gewählt werden. Erfindungsgemäß sind naturgemäß aber solche Verbindungen besonders bevorzugt, in denen alle Variablen eine der bevorzugten und insbesondere eine der besonders bevorzugten Definitionen haben.

Dentalwerkstoffe, die eine Verbindung der Formel I enthalten, in der Y eine acide Gruppe ist, eignen sich besonders als selbstätzende Werkstoffe, insbesondere als Adhäsive, Zemente, und Fissurenversiegler. Bevorzugte acide Harnstoffderivate sind Verbindungen, in denen die Variablen der Formel I die folgenden Bedeutungen haben:
- R¹ =: ein aliphatischer C₂-C₁₂-Rest ist, der durch -O- oder -CO-O- unterbrochen sein kann,
- R² =: ein aliphatischer C₁-C₁₀-Rest, der durch -O- oder -CO-O-unterbrochen sein kann,
- X =: CH₂=CR³-CO-Z- oder R⁴O-CO-C(=CH₂)-CH₂-Z-, wobei Z O oder NR⁵ ist, R³ H oder CH₃ ist und R⁴ und R⁵ unabhängig voneinander jeweils H oder C₁-C₃-Alkyl sind,
- Y =: -PO(OH)₂, -O-PO(OH)₂, -SO₃H,
- n =: 1 oder 2,
- m =: 1 oder 2.

Besonders bevorzugte sind Dentalwerkstoffe dieser Ausführungsform, in denen die Variablen die folgenden Bedeutungen haben:
- R¹, R² =: unabhängig voneinander jeweils ein linearer aliphatischer C₂-C₁₀-Rest, der durch 1 oder 2 -O-unterbrochen sein kann, wobei R¹ und R² vorzugsweise gleich sind,
- X =: CH₂=CR³-CO-Z-, wobei Z O oder NR⁵ ist, oder R⁴O-CO-C(=CH₂)-CH₂-Z-, wobei Z = O ist, R³ H oder CH₃ ist, R⁴ Methyl oder Ethyl ist und R⁵ H, Methyl oder Ethyl ist,
- Y =: -PO(OH)₂, -O-PO(OH)₂, -SO₃H,
- n, m =: jeweils 1.

Gemäß einer weiteren Ausführungsform ist Y eine radikalisch polymerisierbare Gruppe. In solchen Fällen haben die Harnstoffderivate der Formel I vernetzende Eigenschaften. Dentalwerkstoffe, die solche vernetzenden Derivate enthalten, eignen sich besonders als Adhäsive, Beschichtungsmaterialien, Zemente oder Füllungsmaterialien (Komposite), insbesondere als Adhäsive und Zemente. Bevorzugte vernetzende Harnstoffderivate der Formel I sind Verbindungen, in denen die Variablen die folgenden Bedeutungen haben:
- R¹ =: ein aliphatischer C₂-C₁₂-Rest, der durch -O- oder -CO-O-unterbrochen sein kann,
- R² =: ein aliphatischer C₁-C₁₀-Rest, der durch -O- oder -CO-O-unterbrochen sein kann,
- X, Y =: unabhängig voneinander jeweils CH₂=CR³-CO-Z- oder R⁴O-CO-C (=CH₂) -CH₂-Z-, wobei Z O oder NR⁵ ist, R³ H oder CH₃ ist und R⁴ und R⁵ unabhängig voneinander jeweils H oder C₁-C₃-Alkyl sind, wobei X und Y vorzugsweise gleich sind,
- n =: 1 oder 2,
- m =: 1 oder 2; besonders bevorzugt:
- R¹, R² =: unabhängig voneinander jeweils ein linearer aliphatischer C₂-C₁₀-Rest, der durch 1 oder 2 -O-unterbrochen sein kann, wobei R¹ und R² vorzugsweise gleich sind,
- X, Y =: unabhängig voneinander jeweils CH₂=CR³-CO-Z-, wobei Z O oder NR⁵ ist, oder R⁴O-CO-C(=CH₂)-CH₂-Z-, wobei Z = O ist, R³ H oder CH₃ ist und R⁴ Methyl oder Ethyl ist und R⁵ H, Methyl oder Ethyl ist, wobei X und Y vorzugsweise gleich sind,
- n, m =: jeweils 1.

Harnstoffderivate, in denen Y eine acide Gruppe und n größer als 1 ist, haben neben der selbstätzenden und haftungsfördernden Wirkung der aciden Gruppe auch vernetzende Eigenschaften und können als Vernetzer eingesetzt werden. Vorteilhaft können jedoch auch acide Harnstoffderivate der Formel I mit nur einer polymerisierbaren Gruppe mit nicht aciden Harnstoffderivaten der Formal I kombiniert werden, die zwei oder mehr polymerisierbare Gruppen aufweisen. Zusammensetzungen, die gleichzeitig vernetzende und acide Harnstoffderivate der Forml I enthalten, sind erfindungsgemäß besonders bevorzugt.

Die polymerisationsfähigen harnstoffgruppenhaltigen Monomeren der allgemeinen Formel I können einfach hergestellt werden. Beispielsweise lassen sich NH₂-funktionalisierte polymerisationsfähige (Meth)acrylate oder (Methacryl)amides sowie NH₂-funktionalisierte Phosphonate, Phosphate oder Sulfonate mit Isocyanat-funktionalisierten polymerisationsfähigen (Meth)-acrylaten oder (Methacyl)amiden umsetzen, wobei im Falle der Phosphonate, Phosphate oder Sulfonate daran anschließend die Freisetzung der Säuregruppen erfolgt und sich die Verbindungen der allgemeinen Formel I bilden. Die NH₂-funktionalisierte polymerisationsfähige (Meth)acrylate bzw. (Methacryl)amides lassen sich durch (Meth)acrylierung von entsprechenden OH-geschützten Aminoalkoholen und nachfolgende Abspaltung der Schutzgruppe bzw. von entsprechenden Diaminen herstellen. NH₂-funktionalisierte Phosphonate sind beispielsweise ausgehend von α,ω-Dihalogenalkanen durch Arbuzov-Reaktion an einem Molekülende und nachfolgender Gabriel-Reaktion am zweiten Halogenatom zugänglich. Isocyanat-funktionalisierte polymerisationsfähige (Meth)acrylate oder (Methacyl)amide sind durch Umsetzung einsprechender Bromalkyl(meth)acrylate bzw. -(meth)-acrylamide mit Alkalicyanaten herstellbar (vgl. C. Dubosclard-Gottardi, P. Caubere, Y Fort Tetrahedron 51 (1995) 2561-2572):

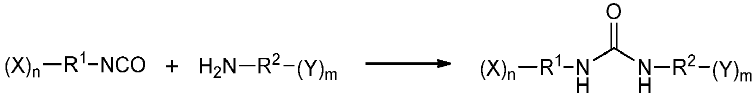

Konkret können Verbindungen der Formel I, in denen X und Y radikalisch polymerisierbare Gruppen sind, z.B. durch Umsetzung von 2-Isocyanatoethylmethacrylat mit 5-Aminopentylmethacrylamid erhalten werden:

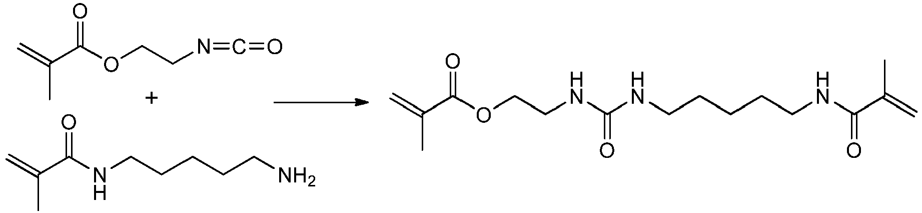

Harnstoffderivate, in denen Y eine acide Gruppe ist, können beispielsweise durch Umsetzung von 2-Isocyanatobutylmethacrylat mit 5-Aminopentylphosphonsäurediethylester und anschließende Freisetzung der Phosphonsäuregruppe hergestellt werden:

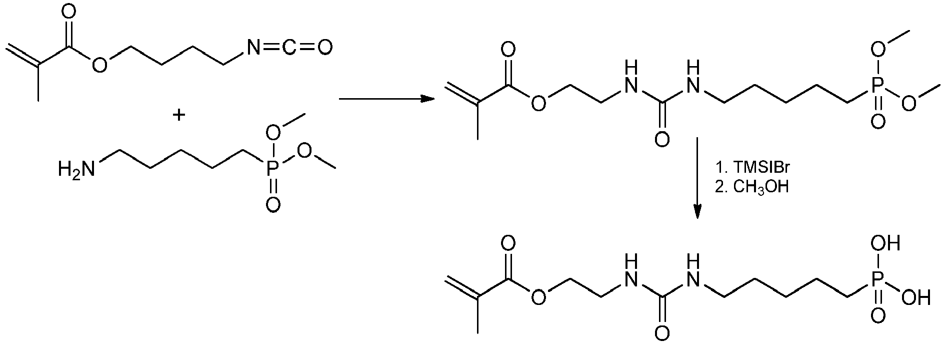

Bevorzugte Beispiele für die erfindungsgemäßen polymerisationsfähigen harnstoffgruppenhaltigen Monomeren der allgemeinen Formel I sind:

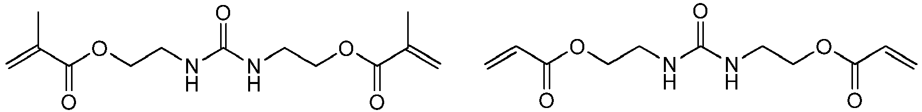

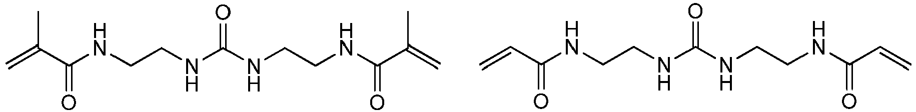

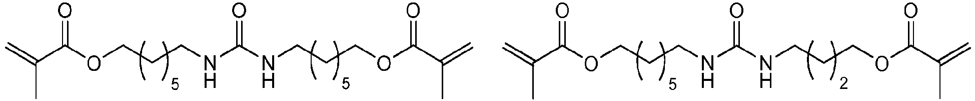

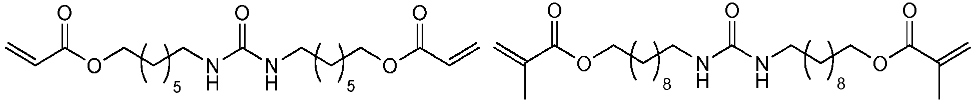

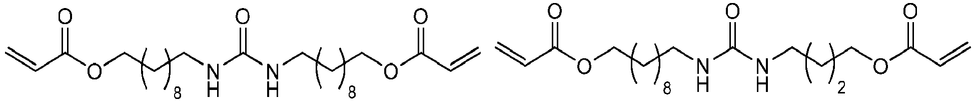

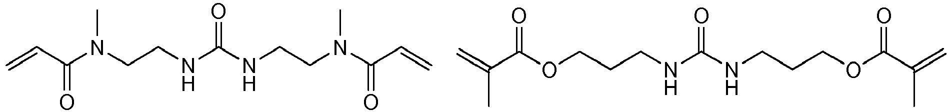

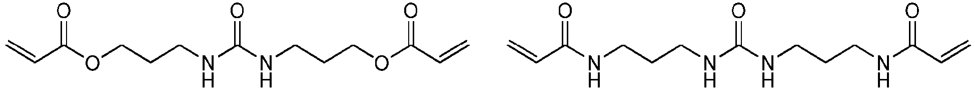

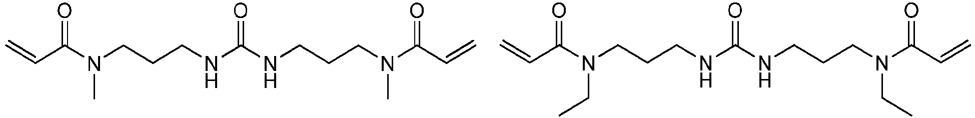

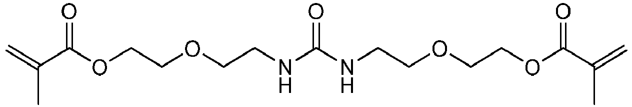

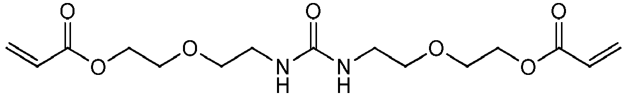

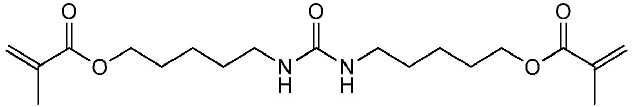

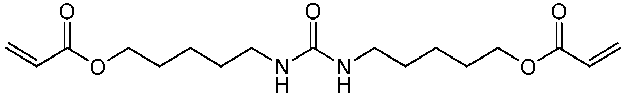

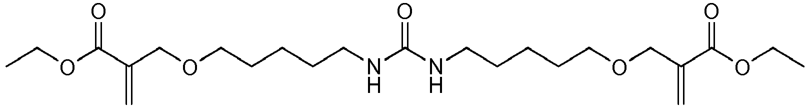

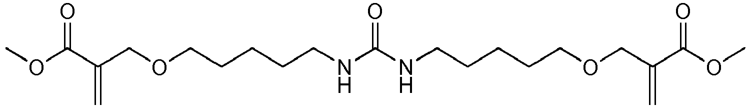

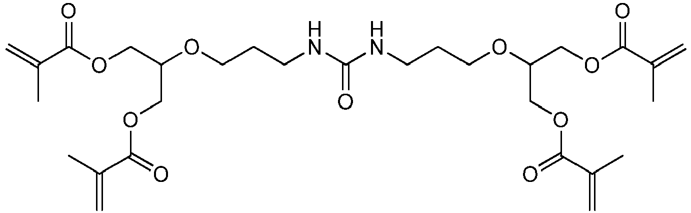

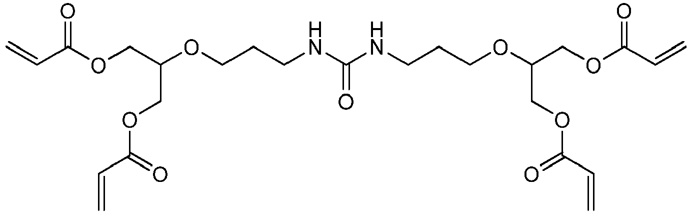

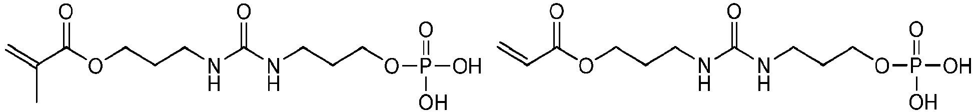

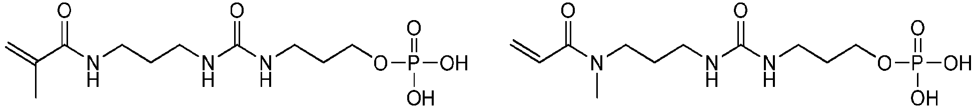

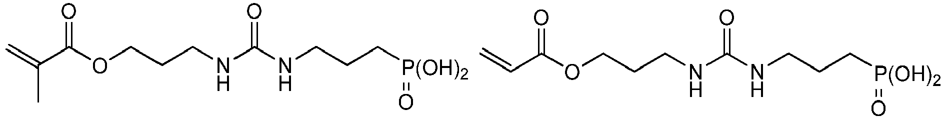

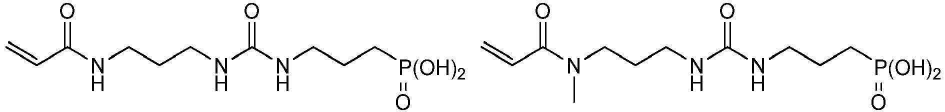

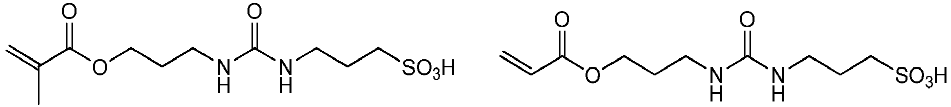

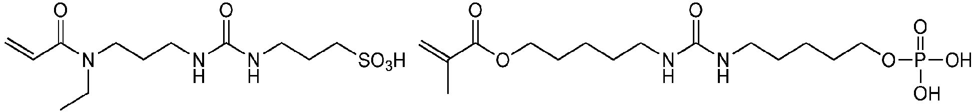

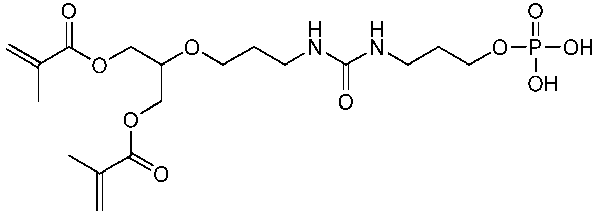

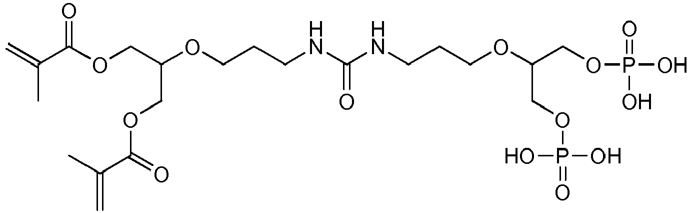

Die polymerisationsfähigen Harnstoffderivate der allgemeinen Formel I eignen sich besonders zur Herstellung von Dentalwerkstoffen, insbesondere von Dentalwerkstoffen mit selbstätzenden Eigenschaften, wie Adhäsiven und Zementen, Kompositen und Beschichtungsmaterialien. Sie können als Haft- und/oder Vernetzerkomponente in Dentalmaterialien verwendet werden. Sie sind sehr gut in Alkoholen, wie z.B. Ethanol und Isopropanol, und in Aceton oder in wässrigen Mischungen davon löslich.

Es wurde überraschend gefunden, dass Lösungen der erfindungsgemäßen Harnstoffderivate der Formel I nicht gelieren, die Harnstoffderivate zeichnen sich durch eine sehr gute radikalische Polymerisationsfähigkeit aus. Die aciden Monomere (Y = acide Gruppe) ergeben zudem sehr gute Haftwerte an Dentin und insbesondere an Zahnschmelz.

Die Harnstoffderivate der Formel I werden vorzugsweise in einer Menge von 0,1 bis 50 Gew.-%, besonders bevorzugt 1 bis 40 Gel.-% und ganz besonders bevorzugt in einer Menge von 2 bis 30 Gew.-% bezogen auf die Gesamtmasse des Dentalwerkstoffs eingesetzt.

Die erfindungsgemäßen Dentalwerkstoffe enthalten vorzugsweise weitere radikalisch polymerisierbare Monomere (Comonomere), besonders bevorzugt mono- oder polyfunktionelle (Meth)acrylsäurederivate. Unter monofunktionellen Monomeren werden Monomere mit einer, unter polyfunktionellen Monomeren mit zwei oder mehr, vorzugsweise zwei bis vier radikalisch polymerisierbaren Gruppen verstanden. Diesbezügliche Beispiele sind Methyl-, Ethyl-, Hydroxyethyl-, Butyl-, Benzyl-, Tetrahydrofurfuryl- oder Isobornyl(meth)acrylat, Bisphenol-A-di(meth)acrylat, Bis-GMA (ein Additionsprodukt aus Methacrylsäure und Bisphenol-A-diglycidylether), UDMA (ein Additionsprodukt aus 2-Hydroxyethylmethacrylat (HEMA) und 2,2,4-Trimethylhexamethylendiisocyanat), Di-, Tri- oder Tetraethylenglycoldi(meth)acrylat, Trimethylolpropantri(meth)-acrylat, Pentaerythrittetra(meth)acrylat, sowie Glycerin-di(meth)acrylat, 1,4-Butandioldi(meth)acrylat, 1,10-Decandiol-di(meth)acrylat oder 1,12-Dodecandioldi(meth)acrylat.

Vorzugsweise wird eine Mischung der genannten Comonomere eingesetzt. Besonders bevorzugt ist eine Mischungen, die 2-Hydroxyethylmethacrylat oder 2-Hydroxypropylmethacrylat in Mischung mit Bis-GMA und/oder UDMA, Triethylenglycoldimethacrylat oder Decandioldimethacrylat enthält.

Weiter bevorzugte Comonomere sind N-mono- oder -disubstitiuierte Acrylamide, wie z.B. N-Ethylacrylamid, N,N-Dimethacrylamid, N-(2-Hydroxyethyl)acrylamid oder N-Methyl-N-(2-hydroxyethyl)acrylamid, bzw. N-monosubstituierte Methacrylamide, wie z.B. N-Ethylmethacrylamid oder N-(2-Hydroxy-ethyl)methacrylamid sowie N-Vinylpyrrolidon oder Allylether einsetzen. Diese Monomere zeichnen sich durch eine hohe Hydrolysestabilität und eine relativ geringe Viskosität aus und eignen sich daher beispielsweise als Verdünnermonomere.

Ebenso bevorzugte Comonomere sind vernetzende Pyrrolidone, wie z.B. 1,6-Bis(3-vinyl-2-pyrrolidonyl)-hexan, oder kommerziell zugängliche Bisacrylamide, wie Methylen- oder Ethylenbisacrylamid, bzw. Bis(meth)acrylamide, wie z.B. N,N'-Diethyl-1,3-bis(acrylamido)-propan, 1,3-Bis(methacrylamido)-propan, 1,4-Bis(acrylamido)-butan oder 1,4-Bis(acryloyl)-piperazin, die durch Umsetzung aus den entsprechenden Diaminen mit (Meth)acrylsäurechlorid synthetisiert werden können. Auch diese Monomere zeichnen sich durch eine hohe Hydrolysestabilität aus. Sie enthalten zwei oder mehr radikalisch polymerisierbare Gruppen und eignen sich daher z.B. als Vernetzermonomere.

Schließlich lassen sich auch Mischungen von einem oder mehreren der voranstehend genannten Monomeren mit weiteren radikalisch polymerisierbaren, säuregruppenhaltigen Haftmonomeren verwenden. Geeignete säuregruppenhaltige Monomere sind polymerisationsfähige Carbonsäuren, wie Maleinsäure, Acrylsäure, Methacrylsäure, 2-(Hydroxy-methyl)acrylsäure, 4-(Meth)acryloyloxyethyltrimellitsäureanhydrid, 10-Methacryloyloxydecylmalonsäure, N-(2-Hydroxy-3-methacryloyloxypropyl)-N-phenylglycin bzw. 4-Vinylbenzoesäure. Beispiele für geeignete Phosphonsäuremonomere sind Vinylphosphonsäure, 4-Vinylphenylphosphonsäure, 4-Vinylbenzylphosphonsäure, 2-Methacryloyloxyethylphosphonsäure, 2-Methacrylamidoethylphosphonsäure, 4-Methacrylamido-4-methyl-pentyl-phosphonsäure, 2-[4-(Dihydroxyphosphoryl)-2-oxa-butyl]-acrylsäure oder 2-[4-(Dihydroxyphosphoryl)-2-oxa-butyl]-acrylsäureethyl- oder -2,4,6-trimethylphenylester. Beispiele für geeignete acide polymerisationsfähige Phosphorsäureester sind 2-Methacryloyloxypropylmono- oder -dihydrogenphosphat, 2-Methacryloyloxyethylmono- oder -dihydrogenphosphat, 2-Methacryloyloxyethylphenyl-hydrogenphosphat, Dipentaerythritolpentamethacryloyloxyphosphat, 10-Methacryloyloxydecyl-dihydrogenphosphat, Phosphorsäuremono-(1-acryloyl-piperidin-4-yl)-ester, 6-(Methacrylamido)hexyldihydrogenphosphat und 1,3-Bis-(N-acryloyl-N-propyl-amino)-propan-2-yl-dihydrogenphosphat. Beispiele für geeignete polymerisationsfähige Sulfonsäuren sind Vinylsulfonsäure, 4-Vinylphenylsulfonsäure oder 3-(Methacrylamido)propylsulfonsäure.

Acide Harnstoffderivate der Formel I werden vorzugsweise mit nicht aciden Comonomeren kombiniert und nicht acide Harnstoffderivate der Formel I mit aciden Comonomeren oder vorzugsweise mit einer Mischung aus aciden und nicht aciden Comonomeren. Bei Mischungen von aciden Harnstoffderivaten der Formel I und nicht aciden Comonomeren liegt der Anteil an nicht aciden Comonomeren vorzugsweise im Bereich von 10 bis 300 Gew.-% bezogen auf die Masse des oder der aciden Harnstoffderivate. Bei Mischungen von nicht aciden Harnstoffderivaten der Formel I und aciden Comonomeren liegt der Anteil an aciden Comonomeren vorzugsweise im Bereich von 5 bis 100 Gew.-% bezogen auf die Summe der Massen an nicht aciden Harnstoffderivaten und ggf. an nicht aciden Comonomeren.

Werden acide Harnstoffderivate der Formel I und acide Comonomere zusammen eingesetzt, enthält diese Mischung vorzugsweise überwiegend und besonders bevorzugt ausschließlich acide Harnstoffderivate der Formel I.

Werden vernetzende Harnstoffderivate der Formel I und vernetzende Comonomere zusammen eingesetzt, enthält diese Mischung vorzugsweise überwiegend und besonders bevorzugt ausschließlich vernetzende Harnstoffderivate der Formel I.

Zur Initiierung der radikalischen Polymerisation enthalten die erfindungsgemäßen Dentalwerkstoffe vorzugsweise einen Initiator für die radikalische Polymerisation. Für die Photopolymerisation werden vorzugsweise Benzophenon, Benzoin sowie deren Derivate oder α-Diketone oder deren Derivate, wie 9,10-Phenanthrenchinon, 1-Phenyl-propan-1,2-dion, Diacetyl oder 4,4'-Dichlorbenzil, eingesetzt. Bevorzugt werden Campherchinon und 2,2-Dimethoxy-2-phenyl-acetophenon und besonders bevorzugt α-Diketone in Kombination mit Aminen als Reduktionsmittel, wie z.B. 4-(Dimethylamino)-benzoesäureester, N,N-Dimethylaminoethylmethacrylat, N,N-Dimethyl-sym.-xylidin oder Triethanolamin, verwendet. Besonders geeignet sind auch Norrish-Typ-I-Photoinitiatoren, vor allem Acyl- der Bisacylphosphinoxide, Monoacyltrialkyl- bzw. Diacyldialkylgermanium-Verbindungen, wie z.B. Benzoyltrimethylgermanium, Dibenzoyldiethylgermanium oder Bis(4-methoxybenzoyl)diethylgermanium. Dabei lassen sich auch Mischungen der verschiedenen Photoinitiatoren einsetzen, wie z.B. Dibenzoyldiethylgermanium in Kombination mit Campherchinon und 4-Dimethylaminobenzoesäureethylester.

Als Initiatoren für eine bei Raumtemperatur durchgeführte Polymerisation werden Redox-Initiatorkombinationen, wie z.B. Kombinationen von Benzoylperoxid mit N,N-Dimethyl-sym.-xylidin, N,N-Dimethyl-p-toluidin, N,N-Diethyl-3,5-di-tert-Butylanilin oder N,N-Diethanol-p-toluidin, verwendet. Darüber hinaus sind auch Redoxsysteme bestehend aus Peroxiden bzw Hydroperoxiden und Reduktionsmitteln, wie z.B. Ascorbinsäure, Barbiturate, Thioharnstoffe oder Sulfinsäuren, besonders geeignet.

Die erfindungsgemäßen Dentalwerkstoffe enthalten vorzugsweise einen Photoinitiator oder eine Kombination aus einem Photoinitiator und einem Redoxinitiator, vorzugweise einem Peroxid. Eine besonders vorteilhafte Initiatorkombination für die duale Härtung ist eine Mischung aus Campherchinon und Benzoylperoxid, wobei auch diese Initiatoren bevorzugt mit einem Amin kombiniert werden.

Weiterhin enthalten die erfindungsgemäß eingesetzten Zusammensetzungen zur Verbesserung der mechanischen Eigenschaften oder zur Einstellung der Viskosität vorzugsweise organische oder anorganische Füllstoffpartikel. Füllstoffe zur Anpassung der mechanischen Eigenschaften haben vorzugsweise einen mittleren Partikeldurchmesser von 10 nm bis 10 µm, bevorzugt von 10 nm bis 1,0 µm, Füllstoffe zur Einstellung der Viskosität vorzugsweise von 10 bis 1000 nm, bevorzugt von 10 bis 200 nm. Diese Füllstoffarten werden vorzugsweise gemeinsam eingesetzt. Wenn nicht anders angegeben, handelt es sich bei dem mittleren Partikeldurchmesser um den massengemittelten Durchschnittswert.

Bevorzugte anorganische partikuläre Füllstoffe sind amorphe kugelförmige Materialien auf der Basis von Oxiden, wie ZrO₂ und TiO₂ bzw. Mischoxiden aus SiO₂, ZrO₂ und/oder TiO_{2,} nanopartikuläre oder mikrofeine Füllstoffe, wie pyrogene Kieselsäure oder Fällungskieselsäure sowie Minifüllstoffe, wie Quarz-, Glaskeramik- oder Glaspulver mit einem mittleren Partikeldurchmesser von 0,01 bis 1 µm sowie röntgenopake Füllstoffe, wie Ytterbiumtrifluorid oder nanopartikuläres Tantal(V)-oxid bzw. Bariumsulfat. Bevorzugte organische Füllstoffe sind Füllstoffe auf der Basis von Poly(meth)-acrylaten, wie z.B. PMMA, oder Cellulosederivaten, wie z.B. Carboxymethylcellulose, die nach dem Härten auf die oben genannte Partikelgröße gemahlen werden. Die organischen Füllstoffe können ihrerseits mit den genannten anorganischen Füllstoffen gefüllt sein.

Lösungsmittelhaltige Dentalwerkstoffe stellen eine weitere bevorzugte Ausführungsform der Erfindung dar. Hierbei kommen besonders Wasser und polare organische Lösungsmittel wie Aceton, Isopropanol und insbesondere Ethanol sowie Mischungen dieser Lösungsmittel in Betracht. Besonders bevorzugt sind Mischungen aus Wasser und polaren organischen Lösungsmitteln, insbesondere Mischungen aus Wasser und Ethanol, Wasser und Aceton oder Wasser, Ethanol und Aceton. Lösungsmittelhalitge Dentalwerkstoffe eigenen sich besonders zur Anwendung als Adhäsive, Fissurenversiegler oder Beschichtungsmaterialien. Adhäsive enthalten vorzugsweise Isopropanol, Ethanol und/oder Aceton bzw. Mischungen der genannten Lösungsmittel mit Wasser. Für Fissurenversiegler oder Be-schichtungsmaterialien werden bevorzugt nur die polaren organsichen Lösungsmittel - ohne Wasser - verwendet.

Gegebenenfalls können die erfindungsgemäß eingesetzten Zusammensetzungen weitere Additive enthalten wie z.B. Stabilisatoren, Aromastoffe, Farbmittel, mikrobizide Wirkstoffe, fluoridionenabgebende Additive, optische Aufheller, Weichmacher und/oder UV-Absorber.

Die erfindungsgemäßen Dentalmaterialien auf der Basis der polymerisationsfähigen harnstoffgruppenhaltigen Monomeren weisen vorzugsweise die folgende Zusammensetzung auf:
a) 0,1 bis 50 Gew.-%, bevorzugt 1 bis 40 Gew.-% und besonders bevorzugt 2 bis 30 Gew.-% polymerisationsfähiges harnstoffgruppenhaltiges Monomer der allgemeinen Formel I,
b) 0,01 bis 10 Gew.-%, besonders bevorzugt 0,1 bis 3,0 Gew.-% Initiator,
c) 0 bis 80 Gew.-%, bevorzugt 0 bis 60 Gew.-% und besonders bevorzugt 5 bis 50 Gew.-% an einem oder mehreren anderen Monomeren,
d) 0 bis 80 Gew.-% Füllstoff,
e) ggf. 0 bis 10 Gew.-%, bevorzugt 0,1 bis 3 Gew.-% Additiv, und
f) 0 bis 70 Gew.-%, bevorzugt 0 bis 60 Gew.-% und besonders bevorzugt 0 bis 50 Gew.-% Lösungsmittel.

Zur Herstellung von Adhäsiven können acide Monomre der Formel I mit nicht aciden Comonomeren kombiniert werden, nicht acide Monomere der Formel I mit aciden Comonomeren und acide Monomere der Formel I mit nicht aciden Monomeren der Formel I.

Dentalmaterialien zur Verwendung als Adhäsiv enthalten bevorzugt die folgenden Komponenten:
a) 0,1 bis 50 Gew.-%, bevorzugt 1 bis 40 Gew.-% und besonders bevorzugt 2 bis 30 Gew.-% Harnstoffderivat der allgemeinen Formel I, wobei Y eine acide Gruppe ist,
b) 0,01 bis 10 Gew.-%, besonders bevorzugt 0,1 bis 3,0 Gew.-% Initiator,
c) 0 bis 80 Gew.-%, bevorzugt 0 bis 60 Gew.-% und besonders bevorzugt 5 bis 50 Gew.-% an einem oder mehreren anderen Monomeren,
d) 0 bis 20 Gew.-% Füllstoff,
e) ggf. 0 bis 10 Gew.-%, bevorzugt 0,1 bis 3 Gew.-% Additiv, und
f) 0 bis 70 Gew.-%, bevorzugt 6 bis 60 Gew.-% und besonders bevorzugt 10 bis 50 Gew.-% Lösungsmittel, vorzugsweise an Wasser und/oder Ethanol, Isopropanol oder Aceton.

Gemäß einer alternativen Ausführungform enthalten die Adhäsive vorzugsweise die folgenden Komponenten:
a) 0,1 bis 50 Gew.-%, bevorzugt 1 bis 40 Gew.-% und besonders bevorzugt 2 bis 30 Gew.-% Harnstoffderivat der allgemeinen Formel I, wobei Y eine polymerisierbare Gruppe ist,
b) 0,01 bis 10 Gew.-%, besonders bevorzugt 0,1 bis 3,0 Gew.-% Initiator,
c1) 0,1 bis 50 Gew.-%, bevorzugt 1 bis 40 Gew.-% und besonders bevorzugt 2 bis 30 Gew.-% an einem oder mehreren anderen aciden Monomeren,
c2) 0 bis 80 Gew.-%, bevorzugt 0 bis 60 Gew.-% und besonders bevorzugt 5 bis 50 Gew.-% an einem oder mehreren anderen nichtaciden Monomeren,
d) 0 bis 20 Gew.-% Füllstoff,
e) ggf. 0 bis 10 Gew.-%, bevorzugt 0,1 bis 3 Gew.-% Additiv, und
f) 0 bis 70 Gew.-%, bevorzugt 6 bis 60 Gew.-% und besonders bevorzugt 10 bis 50 Gew.-% Lösungsmittel, vorzugsweise an Wasser und/oder Ethanol, Isopropanol oder Aceton.

Gemäß einer weiteren alternativen Ausführungform enthalten die Adhäsive vorzugsweise die folgenden Komponenten:
a1) 0,1 bis 50 Gew.-%, bevorzugt 1 bis 40 Gew.-% und besonders bevorzugt 2 bis 30 Gew.-% Harnstoffderivat der allgemeinen Formel I, wobei Y eine acide Gruppe ist,
a2) 0,1 bis 50 Gew.-%, bevorzugt 1 bis 40 Gew.-% und besonders bevorzugt 2 bis 30 Gew.-% Harnstoffderivat der allgemeinen Formel I, wobei Y eine polymerisierbare Gruppe ist,
b) 0,01 bis 10 Gew.-%, besonders bevorzugt 0,1 bis 3,0 Gew.-% Initiator,
c) 0 bis 80 Gew.-%, bevorzugt 0 bis 60 Gew.-% und besonders bevorzugt 5 bis 50 Gew.-% an einem oder mehreren anderen Monomeren,
d) 0 bis 20 Gew.-% Füllstoff,
e) ggf 0 bis 10 Gew.-%, bevorzugt 0,1 bis 3 Gew.-% Additiv, und
f) 0 bis 70 Gew.-%, bevorzugt 6 bis 60 Gew.-% und besonders bevorzugt 10 bis 50 Gew.-% Lösungsmittel, vorzugsweise an Wasser und/oder Ethanol, Isopropanol oder Aceton.

Dentalmaterialien zur Verwendung als Zement oder Füllungsmaterial enthalten bevorzugt die folgenden Komponenten:
a) 0,1 bis 50 Gew.-%, bevorzugt 1 bis 40 Gew.-% und besonders bevorzugt 2 bis 30 Gew.-% Harnstoffderivat der allgemeinen Formel I, wobei Y eine polymerisierbare Gruppe ist,
b) 0,01 bis 10 Gew.-%, besonders bevorzugt 0,1 bis 3,0 Gew.-% Initiator,
c) 0 bis 80 Gew.-%, bevorzugt 0 bis 60 Gew.-% und besonders bevorzugt 5 bis 50 Gew.-% an einem oder mehreren anderen Monomeren,
d) 20 bis 80 Gew.-% Füllstoff, und ggf.
e) 0 bis 10 Gew.-%, bevorzugt 0,1 bis 3 Gew.-% Additiv.

Dentalmaterialien zur Verwendung als Beschichtungsmaterial enthalten bevorzugt die folgenden Komponenten:
a) 0,1 bis 50 Gew.-%, bevorzugt 1 bis 40 Gew.-% und besonders bevorzugt 2 bis 30 Gew.-% Harnstoffderivat der allgemeinen Formel I, wobei Y eine polymerisierbare Gruppe ist,
b) 0,01 bis 10 Gew.-%, besonders bevorzugt 0,1 bis 3,0 Gew.-% Initiator,
c) 0 bis 80 Gew.-%, bevorzugt 0 bis 60 Gew.-% und besonders bevorzugt 5 bis 50 Gew.-% an einem oder mehreren anderen Monomeren,
d) 0 bis 80 Gew.-% Füllstoff,
e) ggf. 0 bis 10 Gew.-%, bevorzugt 0,1 bis 3 Gew.-% Additiv, und
f) 0 bis 70 Gew.-%, bevorzugt 6 bis 60 Gew.-% und besonders bevorzugt 10 bis 50 Gew.-% Lösungsmittel, vorzugsweise Ethanol, Isopropanol und/ oder Aceton.

Dentalmaterialien zur Verwendung als Fissurenversiegler enthalten bevorzugt die folgenden Komponenten:
a) 0,1 bis 50 Gew.-%, bevorzugt 1 bis 40 Gew.-% und besonders bevorzugt 2 bis 30 Gew.-% Harnstoffderivat der allgemeinen Formel I, wobei Y eine polymerisierbare Gruppe ist,
b) 0,01 bis 10 Gew.-%, besonders bevorzugt 0,1 bis 3,0 Gew.-% Initiator,
c) 0 bis 80 Gew.-%, bevorzugt 0 bis 60 Gew.-% und besonders bevorzugt 5 bis 50 Gew.-% an einem oder mehreren anderen Monomeren,
d) 0 bis 60 Gew.-% Füllstoff,
e) ggf. 0 bis 10 Gew.-%, bevorzugt 0,1 bis 3 Gew.-% Additiv und
f) 0 bis 70 Gew.-%, bevorzugt 0 bis 60 Gew.-% und besonders bevorzugt 0 bis 50 Gew.-% Lösungsmittel, vorzugsweise Ethanol, Isopropanol und/ oder Aceton.

Besonders bevorzugt sind solche Dentalwerkstoffe, welche aus den genannten Komponenten bestehen.

Wenn nicht anders angegeben beziehen sich alle Prozentangaben auf die Gesamtmasse der Zusammensetzung. Die Initiatormenge schließt alle Initiatorkomponenten ein, wie z.B. die Masse des eigentlichen Initiators, des Reduktionsmittels etc.

Die erfindungsgemäßen Dentalwerkstoffe eignen sich besonders zur intraoralen Anwendung durch den Zahnarzt zur Restauration geschädigter Zähne (klinische Dentalwerkstoffe). Sie können aber auch extraoral eingesetzt werden, beispielsweise bei der Herstellung oder Reparatur von Dentalrestaurationen (technische Dentalwerkstoffe).

Die Erfindung wird im Folgenden anhand von Beispielen näher erläutert.

### Ausführungsbeispiele

### Beispiel 1:

### Synthese von 2-[(2-Methacryloyloxyethylamino)carbonylamino]-ethylphosphonsäure (MEHEPA)

### a) Synthese von 2-[(2-Methacryloyloxyethylamino) carbonylamino]-ethylphosphonsäurediethylester 1

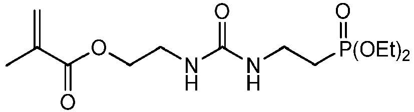

2-Isocyanatoethylmethacrylat (4.7 ml, 33.3 mmol, 1.0 Äquivalent) wurde unter Rühren tropfenweise zu einer Lösung des 2-Aminoethylphosphonsäurediethylester (33.3 mmol), der analog der Literatur (Gali, H.; Prabhu, K. R.; Karra, S. R.; Katti, K. V. J. Org. Chem. 2000, 65, 676-680) hergestellt wurde, in wasserfreiem Methylenchlorid (70 ml) bei 0 °C zugegeben. Die Reaktionsmischung wurde 15 min bei 0 °C für 2 h bei Raumtemperatur gerührt. Das Reaktionsprodukt wurde im Vakuum eingeengt und das erhaltene Rohprodukt wurde durch Flash-Säulenchromatographie (Eluent: Ethylacetat/Methanol: 90/10) gereinigt und ergab ein farbloses Öl; Ausbeute: 98 %.
¹H-NMR (400 MHz, CDCl₃, ppm) : δ = 1.28 (t, ³J_{HH} = 7.1 Hz, 6H, POCH₂CH₃) ; 1.90 (s, 3H, CH₃) ; 1.97 (dt, ³J_{HH} = 6.6 Hz, ²J_{HP} = 17.3 Hz, 2H, CH₂P); 3.36-3.49 (m, 4H, CH₂N); 3.96-4.10 (m, 4H, POCH₂CH₃); 4.16 (t, ³J_{HH} = 5.6 Hz, 2H, OCH₂CH₂NH); 5.46 (t, ³J_{HH} = 5.8 Hz, 1H, NH) ; 5.52-5.55 (m, 1H, C=CH₂) ; 5. 75 (t, ³J_{HH} = 5.8 Hz, 1H, NH) ; 6.08 (s, 1H, C=CH₂).
¹³C-NMR (101 MHz, CDCl₃, ppm) : δ = 16.4 (d,³J_{CP} = 6.2 Hz, POCH₂CH₃); 18.3 (CH₃); 26.2 (d, ¹J_{CP} = 138.4 Hz, CH₂P); 34.3 (d, ²JCP = 5.2 Hz, NHCH₂CH₂P) ; 39.2 (OCH₂CH₂NH); 61.8 (d, ²J_{CP} = 6.5 Hz, POCH₂CH₃); 64.2 (OCH₂CH₂NH); 125.8 (C=CH₂); 136.1 (C=CH₂); 158.2 (NHC=O); 167.3 (C=O).
³¹P-NMR (162 MHz, CDCl₃, ppm): 30.5.

### b) Synthese von 2-[(2-Methacryloyloxyethylamino)carbonylamino]-ethylphosphonsäure (MEHEPA)

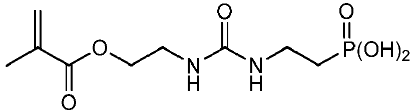

Trimethylsilylbromid (5.9 ml, 44.6 mmol, 3.0 Äquivalente) wurde zu einer Lösung des entsprechenden Phosphonates **1** (14.9 mmol) in wasserfreiem Methylenchlorid (50 ml) gegeben und 5 h bei 30 °C gerührt. Danach wurde das Reaktionsprodukt im Vakuum eingeengt, Methanol (50 ml) zugegeben und 30 min bei Raumtemperatur gerührt. Nach Zugabe von BHT (250 ppm) wurde die Lösung im Feinvakuum bis zur Gewichtskonstanz eingeengt. **MEHEPA** wurde als gelbes hochvisköses Öl erhalten; Ausbeute: 100 %.
¹H-NMR (400 MHz, MeOD, ppm): δ = 1.93 (s, 3H, CH₃); 1.96-2.07 (m, 2H, CH₂P); 3.40-3.50 (m, 4H, CH₂N); 4.21 (t, ³J_{HH} = 5.4 Hz, 2H, OCH₂CH₂NH); 5.63-5.66 (m, 1H, C=CH₂); 6.12-6.15 (m, 1H, C=CH₂).
¹³C-NMR (101 MHz, MeOD, ppm): δ = 18.4 (CH₃) ; 28.9 (d, ¹J_{CP} = 135.8 Hz, CH₂P) ; 36.1 (NHCH₂CH₂P); 40.5 (OCH₂CH₂NH); 64.7 (OCH₂CH₂NH); 126.5 (C=CH₂); 137.5 (C=CH₂); 160.8 (NHC=O); 168.6 (C=O).
³¹P-NMR (162 MHz, MeOD, ppm): 27.1.

### Beispiel 2:

### Synthese von 6-[(2-Methacryloyloxyethylamino)carbonylamino]-hexylphosphonsäure (MEHHPA)

### a) Synthese von 6-Phthalimidohexyl-phosphonsäurediethylester 2

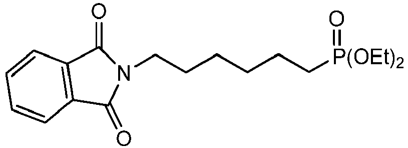

6-Bromhexyl-diethylphosphonat (15.0 g, 49.8 mmol) wurde zu einer Lösung von Kaliumphthalimid (13.8 g, 74.7 mmol, 1.5 Äquivalente) in DMF (100 ml) zugegeben. Die Reaktionsmischung wurde 17 h bei 100 °C gerührt. Nach Filtration wurde die Lösung im Vakuum eingeengt. Zum Rohprodukt wurde Ethylacetat (100 ml) gegeben. Die Lösung wurde filtriert und mit destilliertem Wasser gewaschen (2x100 ml). Danach wurde die organische Phase über wasserfreiem Na₂SO₄ getrocknet und im Vakuum eingeengt. Das erhaltene Rohprodukt wurde durch Flash-Säulenchromatographie (Eluent: Ethylacetat) gereinigt und ergab 13.14 g (35.8 mmol) des Phosphonates **2** als schwach gelbliche Flüssigkeit; Ausbeute: 72 %.
¹H-NMR (400 MHz, CDCl₃, ppm): δ = 1.28-1.48 (m, 4H, CH₂); 1.31 (t, ³J_{HH} = 7.1Hz, 6H, POCH₂CH₃); 1.53-1.77 (m, 6H, CH₂); 3.68 (t, ³J_{HH} = 7.2 Hz, 2H, CH₂N); 4.00-4.15( m, 4H, POCH₂CH₃); 7.67-7.74 (m, 2H, CH_{Ar);} 7.81-7.88 (m, 2H, CH_{Ar}).
¹³C-NMR (101 MHz, CDCl₃, ppm) : δ = 16.4 (d, ³J_{CP} = 6.0 Hz, POCH₂CH₃); 22.3 (d, ²J_{CP} = 5.3 Hz, CH₂CH₂P); 25.6 (d, ¹J_{CP} = 140.6 Hz, CH₂P); 26.3 (CH₂); 28.3 (CH₂); 30.1 (d, ³J_{CP} = 17.1 Hz, CH₂CH₂CH₂P); 37.8 (CH₂N); 61.3 (d, ²J_{CP} = 6.4 Hz, POCH₂CH₃); 123. 1 (CH_{Ar}); 132.1 (C_{Ar}); 133.8 (CH_{Ar}); 168.4 (C=O).
³¹P NMR (162 MHz, CDCl₃, ppm): 32.3.

### b) Synthese von 6-Aminohexyl-phosphonsäurediethylester 3

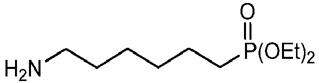

Hydrazinmonohydrat (2.66 g, 53.1 mmol, 1.5 Äquivalente) wurde zu einer Lösung des Phosphonates **2** (13.0 g, 35.4 mmol) in Ethanol (135 ml) zugegeben. Die Reaktionsmischung wurde 2 h unter Rückfluss gerührt und im Vakuum eingeengt. Zum Rohprodukt wurde Natronlauge (10 Gew.-%, 250 ml) zugegeben. Die wässrige Lösung wurde mit Methylenchlorid (3x200 ml) extrahiert. Die organischen Phasen wurden gesammelt und über wasserfreiem Na₂SO₄ getrocknet. Nach Einengen im Vakuum wurden 7.37 g (31.1 mmol) des Amins **3** als farblose Flüssigkeit isoliert; Ausbeute: 88 %.
¹H-NMR (400 MHz, CDCl₃, ppm): δ = 1.25-1.45 (m, 4H, CH₂); 1.28 (t, ³J_{HH} - 7.1 Hz, 6H, POCH₂CH₃) ; 1.51-1.75 (m, 6H, CH₂); 2.64 (t, ³J_{HH} = 7.0 Hz, 2H, CH₂N); 3.98-4.13 (m, 4H, POCH₂CH₃).
¹³C-NMR (101 MHz, CDCl₃, ppm): δ = 16.4 (d, ³J_{CP} = 5.9Hz, POCH₂CH₃); 22.4 (d, ²J_{CP} = 5.4 Hz, CH₂CH₂P); 25.6 (d, ¹J_{CP} = 140.6 Hz, CH₂P); 26.4 (CH₂); 30.4 (d, ³J_{CP} = 16.8 Hz, CH₂CH₂CH₂P); 33.5 (CH₂); 42.1 (CH₂N); 61.3 (d, ²J_{CP} = 6.5Hz, POCH₂CH₃).
³¹P-NMR(162 MHz, CDCl₃, ppm): 32.4.

### c) Synthese von 6-[(2-Methacryloyloxyethylamino)carbonylamino]-hexylphosphonsäurediethylester 4

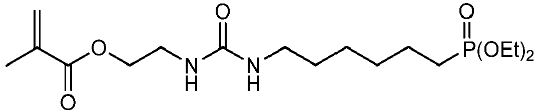

2-Isocyanatoethylmethacrylat (4.7 ml, 33.3 mmol, 1.0 Äquivalent) wurde unter Rühren tropfenweise zu einer Lösung des Aminophosphonats **3** (33.3 mmol) in wasserfreiem Methylenchlorid (70 ml) bei 0 °C zugegeben. Die Reaktionsmischung wurde 15 min bei 0 °C und 2 h bei Raumtemperatur gerührt, wurde im Vakuum eingeengt und das erhaltene Rohprodukt wurde durch Flash-Säulenchromatographie (Eluent: Ethylacetat/Methanol: 90/10) gereinigt und ergab das Monomer **4** als farbloses Öl, Ausbeute: 97 %.
¹H-NMR (400 MHz, CDCl₃, ppm): δ = 1.24-1.49 (m, 6H, CH₂); 1.29 (t, ³J_{HH} = 7.1 Hz, 6H, POCH₂CH₃₎; 1.50-1.62 (m, 2H, CH₂); 1.63-1.75 (m, 2H, CH₂); 1.91 (s, 3H, CH₃); 3.11 (q, ³J_{HH} = 6.8 Hz, 2H, CH₂N); 3.45 (q, ³J_{HH} = 5.6 Hz, 2H, CH₂N); 3.96-4.10 (m, 4H, POCH₂CH₃); 4.17 (t, ³J_{HH} = 5.6 Hz, 2H, OCH₂CH₂NH); 5.22 (t, ³J_{HH} = 5.4 Hz, 1H, NH); 5.32 (t, ³J_{HH} = 5.8 Hz, 1H, NH) ; 5.53-5.56 (m, 1H, C=CH₂); 6.09 (s, 1H, C=CH₂).
¹³C-NMR (101 MHz, CDCl₃, ppm): δ = 16.4 (d, ³J_{CP} = 6.1 Hz, POCH₂CH₃); 18.3 (CH₃); 22.2 (d, ²J_{CP} = 5.2 Hz, CH₂CH₂P); 25.2 (d, ¹J_{CP} = 140.4 Hz, CH₂P); 26.2 (CH₂); 29.7 (CH₂); 29.9 (d, ³J_{CP} = 15.8 Hz, CH₂CH₂CH₂P); 39.2 (CH₂N); 40.0 (CH₂N); 61.5 (d, ²J_{CP} = 6.6 Hz, POCH₂CH₃); 64.4 (OCH₂CH₂N); 125.8 (C=CH₂); 136.1 (C=CH₂); 158.5 (NHC=O); 167.4 (C=O).
³¹P-NMR (162 MHz, CDCl₃, ppm): 32.4.

### d) Synthese von 6-[(2-Methacryloyloxyethylamino)carbonylamino]-hexylphosphonsäure (MEHHPA)

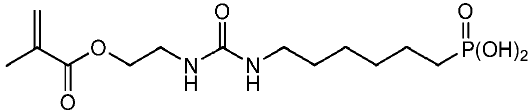

Trimethylsilylbromid (5.9 ml, 44.6 mmol, 3.0 Äquivalente) wurde zu einer Lösung des Phosphonates **4** (14.9 mmol) in wasserfreiem Methylenchlorid (50 ml) zugetropft und 5 h bei 30 °C gerührt. Danach wurde das Reaktionsprodukt im Vakuum eingeengt, Methanol (50 ml) zugegeben und 30 Min bei Raumtemperatur gerührt. Nach Zugabe von BHT (250 ppm) wurde die Lösung im Feinvakuum bis zur Gewichtskonstanz getrocknet und ergab **MEHHPA** als gelbes hochvisköses Öl, Ausbeute: 100 %.
¹H-NMR (400 MHz, MeOD, ppm): δ = 1.32-1.68 (m, 8H, CH₂); 1.69-1.81 (m, 2H, CH₂); 1.94 (s, 3H, CH₃); 3.20 (t, ³J_{HH} = 7.1 Hz, 2H, CH₂N); 3.52 (t, ³J_{HH} = 5.4 Hz, 2H, CH₂N); 4.24 (t, ³J_{HH} = 5.4 Hz, 2H, OCH₂CH₂NH); 5.64-5.67 (m, 1H, C=CH₂); 6.13 (s, 1H, C=CH₂).
¹³C-NMR (101 MHz, MeOD, ppm): δ = 17.1 (CH₃); 22.2 (d, ²J_{CP} = 4.8Hz, CH₂CH₂P); 25.9 (CH₂); 26.2 (d, ¹J_{CP} = 137.7 Hz, CH₂P); 29.0 (CH₂); 29.8 (d, ³J_{CP} = 16.7 Hz, CH₂CH₂CH₂P); 39.4 (CH₂N); 40.4 (CH₂N); 63.0 (OCH₂CH₂N); 125.3 (C=CH₂); 136.1 (C=CH₂); 159.5 (NHC=O); 167.2 (C=O).
³¹P-NMR (162 MHz, MeOD, ppm): 31.6.

### Beispiel 3:

### Synthese von 6-[2-Methacryloyloxyethylamino)carbonylamino]-hexyldihydrogenphosphat (MEHHDPA)

### a) Synthese von 2-[(6-Hydroxyhexylamino)carbonylaminol-ethyl-methacrylat 5

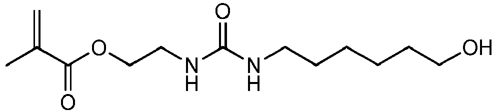

2-Isocyanatoethylmethacrylat (12.1 ml, 85.5 mmol, 1.0 Äquivalent) wurde unter Rühren tropfenweise zu einer Lösung von 6-Aminohexanol (10.0 g, 85.5 mmol) in wasserfreiem Methylenchlorid (150 ml) bei 0 °C zugetropft. Die Reaktionsmischung wurde 15 min bei 0 °C für 2 h bei Raumtemperatur gerührt. Danach wurde im Vakuum eingeengt, dem Produkt Hexan (200 ml) zugegeben und die Mischung 1 h bei Raumtemperatur gerührt. Durch Filtration der Suspension wurde der Feststoff abgetrennt und im Vakuum (0.1 mbar, 1 h) getrocknet. Es ergaben sich 22.74 g (83.6 mmol) des Alkohols **5** als ein weißer Feststoff; Ausbeute: 98%.
¹H-NMR (400 MHz, CDCl₃, ppm): δ = 1.29-1.43 (m, 4H, CH₂); 1.45-1.60 (m, 4H, CH₂); 1.94 (s, 3H, CH₃); 2.08 (s, 1H, OH); 3.15 (q, ³J_{HH} = 6.4 Hz, 2H, CH₂N); 3.49 (q, ³J_{HH} = 5.6 Hz, 2H, OCH₂CH₂NH); 3.62 (t, ³J_{HH} = 6.4 Hz, 2H, CH₂OH); 4.22 (t, ³J_{HH} = 5.6 Hz, 2H, OCH₂CH₂NH); 4.79 (t, ³J_{HH} = 5.5 Hz, 1H, NH); 4.97 (t, ³J_{HH} = 5.7 Hz, 1H, NH); 5.58-5.61 (m, 1H, C=CH₂); 6.12 (s, 1H, C=CH₂).
¹³C-NMR (101 MHz, CDCl₃, ppm): δ = 18.2 (CH₃); 23.4 (CH₂); 26.5 (CH₂); 30.2 (CH₂); 32.5 (CH₂); 39.2 (CH₂N); 40.1 (CH₂N); 62.3 (CH₂OH); 64.3 (OCH₂CH₂N); 126.0 (C=CH₂); 136.0 (C=CH₂); 158.8 (NHC=O); 167.5 (C=O).

### b) Synthese von 6-[(2-Methacryloyloxyethylamino)carbonylamino]-hexyldihydrogenphosphat (MEHHDPA)

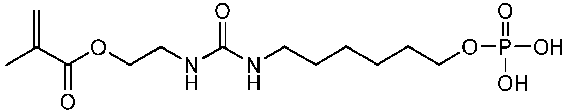

Zu einer auf -10°C abgekühlten Lösung von 14.0 g (91.3 mmol) Phosphoroxychlorid in 200 ml Tetrahydrofuran (THF) wird langsam eine Lösung von 22.6 g (83 mmol) Mononer **5,** 9.24 g (91.3 mmol) Triethylamin und 11 mg BHT in 100 ml THF so zugetropft, dass die Innentemperatur -5°C nicht übersteigt. Nach zweistündigem Rühren bei -10°C werden dem Reaktionsgemisch vorsichtig 13.0 g (721 mmol) Wasser zugegeben und der Ansatz noch 15 min bei 0°C gerührt. Der abgeschiedene Feststoff wird abgesaugt, das Filtrat 3x mit je 200 ml gesättigter Kochsalzlösung gewaschen, die organische Phase mit wasserfreiem Natriumsulfat getrocknet, und anschliessend wird das THF am Rotavapor bei 40°C abdestilliert. Der erhaltene Rückstand wird zum Herausschleppen von restlichem Wasser mit 100 ml Acetonitril versetzt und dieses am Rotavapor bei 40°C wieder abdestilliert. Dieser Vorgang wird noch zweimal wiederholt. Es ergaben sich 30.2 g von **MEHHDPA** als bräunliches, hochviskoses Öl; Ausbeute: 103 % (lt. ¹H-NMR-Spektrum noch 3.5 Gew.-% THF enthalten).
¹H-NMR (400 MHz, DMSO-*d₆*, ppm) : δ = 1.25-1.39 und 1.52-1.58 (2 m, 6H, 2H, CH₂)4); 1.88 (s, 3H, CH₃); 2.97 (t, *J* = 6.9 Hz, 2H, CH₂(CH₂)₄); 3.27 (t, *J* = 5.6 Hz, 2H, NCH₂); 3.78-3.83 (m, 2H, POCH₂); 4.05 (t, *J* = 5.6 Hz, 2H, OCH₂); 5.68 und 6.06 (2s, je 1H, =CH₂); 9.30 (br. s, 2H, OH).
¹³C-NMR (100 MHz, DMSO-*d₆*, ppm) : δ = 18.4 (CH₃); 25.3, 26.4, 30.3 und 30.4 [(CH₂)₄]; 38.7 und 39.7 (NCH₂); 64.6 (OCH₂); 65.7 (d, *²J_{C,P}* = 6.0 Hz, POCH₂); 126.3 (=CH₂); 136.3 (=C); 158.5 (NC=O); 167.0 (C=O).
³¹P-NMR (162 MHz, DMSO-*d₆*, ppm): δ = -1.13.

### Beispiel 4:

### Synthese von N,N'-Bis-(2-methacryloyloxyethyl)-harnstoff (BMAEH)

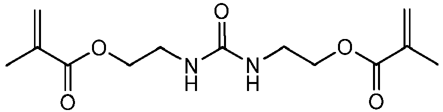

2-Isocyanatoethylmethacrylat (18.2 ml, 129 mmol) und BHT (5.0 mg) wurden in THF (200 mL) gelöst. Dann wurde Wasser (13.9 ml, 774 mmol, 6.0 Äquivalente) zugegeben und die Reaktionsmischung 2 h bei 60 °C gerührt. Nach Einengen der Reaktionsmischung im Vakuum wurde der wässrige Rückstand mit Methylenchlorid extrahiert (3x50 ml), die organischen Phasen gesammelt und über wasserfreiem Na₂SO₄ getrocknet. Das Rohprodukt wurde anschließend mit Hexan (150 ml) versetzt, die Mischung 1 h bei Raumtemperatur gerührt, der Feststoff der Suspension abfiltriert und im Vakuum (0.1 mbar, 1 h) getrocknet. Es ergaben sich 15.48 g (54.5 mmol) der Verbindung **BMAEH** als weißer Feststoff; Ausbeute: 84%. Das Monomer ist in THF (35 Gew.-%) und Methanol (50 Gew.-%) löslich. In beiden Fällen bildeten die konzentrierten Lösungen kein Gel.
¹H-NMR (400 MHz, CDCl₃, ppm): δ = 1.92 (s, 6H, CH₃); 3.48 (q, ³J_{HH} = 5.6 Hz, 4H, CH₂N); 4.21 (t, ³J_{HH} = 6.4 Hz, 4H, CH₂O); 5.00 (t, ³J_{HH} = 5.6 Hz, 2H, NH); 5.56-5.59 (m, 2H, C=CH₂); 6.10 (s, 2H, C=CH₂) .

### Beispiel 5:

### Synthese von N,N'-Bis-2-methacryloxyhexyl -harnstoff (BMAHH)

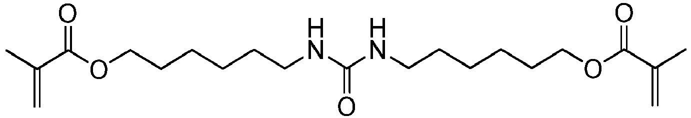

Das Monomer **BMAHH** wurde analog der Literatur (Dubosclard-Gottardi, C.; Caubere, P.; Fort, Y. Tetrahedron 1995, 51, 2561-2572) hergestellt.

### Beispiel 6:

### Untersuchung der Photopolymerisation der Harnstoffmonomeren MEHEPA und MEHHPA mittels DSC

Zu einer Mischung aus dem Vernetzer N,N'-Diethyl-1,3-bis(acrylamido)-propan (**DEPBA**) und **MEHEPA** bzw. **MEHHPA** im Molverhältnis 8:2 wurde 0,1 Gew.-% des Photoinitiators Bis(4-methoxybenzoyl)diethylgermanium zugesetzt. Die Lösung wurde in einem Differential-Scanning-Kalorimeter Diamond (Perkin Elmer) mit Photopolymerisationsaufsatz durch Bestrahlung mit einer LED-Lampe (Bluephase, Ivoclar Vivadent AG) für 2 min bei 37 °C polymerisiert. Die Ergebnisse sind in Tabelle 1 dargestellt. Die Ergebnisse belegen eine sehr gute radikalische Copolymerisationsfähigkeit der harnstoffgruppenhaltigen Phosphonsäuremonomere **MEHEPA** und **MEHHPA,** da sie die Polymerisationsgeschwindigkeit und den Doppelbindungsumsatz nicht gravierend verringern. Dies ist besonders im Hinblick auf die Tatsache bemerkenswert, dass sie nur eine polymerisationsfähige Gruppe aufweisen.

**Tabelle 1**

| **Monomer** | **tₘₐₓ (s)** | **DC (%)** | **R**ₚ**_{*m*ax} (s⁻¹)** |
|---|---|---|---|
| **DEPBA* )** | 2.8 | 62.6 | 0.075 |
| **DEPBA / MEHEPA** | 2.9 | 50.8 | 0.064 |
| (8/2, mol/mol) | | | |
| **DEPBA / MEHHPA** | 3.1 | 53.6 | 0.061 |
| (8/2, mol/mol) | | | |

| | | | |
|---|---|---|---|
| *) Vergleich tₘₐₓ = Zeit bis zum Erreichen der maximalen Polymerisationsgeschwindigkeit DC = Doppelbindungsumsatz Rₚₘₐₓ = maximale Polymerisationsgeschwindigkeit | | | |

### Beispiel 7:

### Untersuchung der Photopolymerisation des N,N'-Bis-(2-methacryl oyloxyethyl)harnstoffs BMAEH mittels DSC

Zu einer Mischung aus dem 2-Hydroxyethylmethacrylat (**HEMA**) und Glycerindimethacrylat (**GDMA**) bzw. **BMAEH** im Molverhältnis 8:2 wurde 0,5 Gew.-% des Photoinitiators Bis(4-methoxybenzoyl)di-ethylgermanium zugesetzt. Die Lösung wurde in einem Differential-Scanning-Kalorimeter Diamond (Perkin Elmer) mit Photopolymerisationsaufsatz durch Bestrahlung mit einer LED-Lampe (Bluephase, Ivoclar Vivadent AG) für 2 min bei 37 °C polymerisiert. Die Ergebnisse sind in Tabelle 2 dargestellt. Es zeigte sich, dass das Monomer **BMAEH** reaktiver als **GDMA** ist.

**Tabelle 2**

| Monomer | **t**ₘₐₓ **(s)** | **DC (%)** | **R**ₚₘₐₓ **(s⁻¹)** |
|---|---|---|---|
| **HEMA / GDMA** | 6.6 | 70.0 | 0.062 |
| (8/2, mol/mol) | | | |
| **HEMA** / **BMAEH** | 5.8 | 69.0 | 0.071 |
| (8/2, mol/mol) | | | |

| | | | |
|---|---|---|---|
| tₘₐₓ = Zeit bis zum Erreichen der maximalen Polymerisationsgeschwindigkeit DC = Doppelbindungsumsatz Rₚₘₐₓ = maximale Polymerisationsgeschwindigkeit | | | |

### Beispiel 8:

### Adhäsive und Haftuntersuchungen auf der Basis von den Säuremonomeren MEHEPA, MEHHPA und MEHHDPA

Zur Untersuchung der Dentin- und Schmelzhaftung auf Rinderzähne wurden durch Mischen der Komponenten Adhäsive mit der in Tabelle 3 angegebenen Zusammensetzung hergestellt. Rinderzähne wurden so in Kunststoffzylinder eingebettet, dass sich das Dentin bzw. der Schmelz und der Kunststoff in einer Ebene befanden. Mit einem Microbrush wurde eine Schicht Adhäsiv obiger Zusammensetzung aufgepinselt, ca. 20 s das Adhäsiv auf der Zahnhartsubstanz bewegt, mit einem Luftgebläse zur Entfernung des Lösungsmittels kurz verblasen und für 10 s mit einer LED-Lampe (Bluephase, Ivoclar Vivadent) belichtet. Auf die Adhäsivschicht polymerisierte man einen Kompositzylinder aus Tetric^{®} EvoCeram (Ivoclar Vivadent) auf. Anschließend wurden die Prüfkörper 24 h bei 37 °C in Wasser gelagert und die Scherhaftfestigkeit entsprechend der ISO-Richtlinie "ISO 2003-ISO TR 11405: Dental Materials Guidance on Testing of Adhesion to Tooth Structure" bestimmt (Tabelle 4). Im Vergleich mit dem 10-Methacryloyloxydecylphosphonsäure, einer Phosphosäure nach dem Stand der Technik, ergaben die neuen Harnstoffmonomere eine deutlich höhere Haftung, insbesondere and Zahnschmelz.

**Tabelle 3: Zusammensetzung der Adhäsive (Angaben in Gew.-%)**

| **Komponente** | **Adhäsiv A** | **Adhäsiv B** | **Adhäsiv C*)** |
|---|---|---|---|
| **MEHEPA** (Bsp. 1) | 15,0 | - | - |
| **MEHHPA** (Bsp. 2) | - | 15,0 | - |
| **MEHHDPA** (Bsp. 3) | - | - | - |
| **MDPA**^{**3**)} | - | - | 15, 0 |
| Bis-GMA¹⁾ | 19,0 | 19,0 | 19,0 |
| DEPBA⁴⁾ | 43,2 | 43,2 | 43,2 |
| Aerosil R709⁵⁾ | 1,4 | 1,4 | 1,4 |
| Photoinitiator²⁾ | 2, 6 | 2, 6 | 2, 6 |
| deionisiertes Wasser | 14, 6 | 14, 6 | 14, 6 |
| Isopropanol | 4,2 | 4,2 | 4,2 |

| | | | |
|---|---|---|---|
| *) Vergleich ¹⁾ Additionsprodukt aus Methacrylsäure und Bisphenol-A-diglycidylether ²⁾ Mischung aus Kampherchinon (0,9%), 4-Dimethyl-benzoesäureethylester (0,4%) und 2,4,6-Trimethylbenzoyldiphenylphosphinoxid (Lucerin TPO, 1,3%) ³⁾ 10-Methacryloyloxydecylphosphonsäure ⁴⁾ N,N'-Diethyl-1,3-bis(acrylamido)-propan ⁵⁾ methacrylsilanisierte pyrogene Kieselsäure mit einer mittleren Primärteilchengröße von 40 nm (Degussa) | | | |

**Tabelle 4: Dentin und Schmelz Haftwerte**

| **Adhäsiv** | **Dentin SBS¹⁾ (MPa)** | **Schmelz SBS¹⁾ (MPa)** |
|---|---|---|
| **A** | 24.5 ± 6.0 (1/5) | 28.5 ± 4.9 |
| **B** | 28.5 ± 1.9 (2/5) | 31.0 ± 3.5 |
| **C*)** | 22.6 ± 3.4 (0/5) | 16.8 ± 4.6; |

| | | |
|---|---|---|
| *) Vergleich ¹⁾ Scherhaftung (shear bond strength) | | |

## Patentansprüche

1. Dentalwerkstoff, **dadurch gekennzeichnet, dass** er ein Harnstoffderivat gemäß der allgemeinen Formel I enthält, 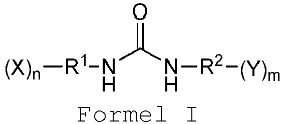 in der
R¹, R² = unabhängig voneinander jeweils ein aliphatischer C₁-C₁₅-Rest sind, der durch -O-, -S-, -CO-O-, unterbrochen sein kann,
X = eine radikalisch polymerisierbare Gruppe ist,
Y = eine radikalisch polymerisierbare Gruppe oder eine acide Gruppe ist,
n, m = unabhängig voneinander jeweils 1, 2, oder 3 sind.

2. Dentalwerkstoff nach Anspruch 1, wobei
X = CH₂=CR³-CO-Z- oder R⁴O-CO-C(=CH₂)-CH₂-Z- ist, wobei Z O oder NR⁵ ist oder entfällt, R³ H oder CH₃ ist und R⁴ und R⁵ unabhängig voneinander jeweils H oder C₁-C₇-Alkyl sind, und /oder
Y = entweder CH2=CR^{3'}-CO-Z'- oder R^{4'}O-CO-C(=CH₂)-CH₂-Z'- ist, wobei Z' O oder NR^{5'} ist oder entfällt, R^{3'} H oder CH₃ ist und R^{4'} und R^{5'} unabhängig voneinander jeweils H oder C₁-C₇-Alkyl sind, oder -PO(OH)₂, -O-PO(OH)₂, -SO₃H ist.

3. Dentalwerkstoff nach Anspruch 1 oder 2, wobei
R¹ = ein aliphatischer C₂-C₁₂-Rest ist, der durch -O- oder -CO-O- unterbrochen sein kann,
R² = ein aliphatischer C₁-C₁₀-Rest ist, der durch -O- oder -CO-O- unterbrochen sein kann,
X = CH₂=CR³-CO-Z- oder R⁴O-CO-C(=CH₂)-CH₂-Z- ist, wobei Z O oder NR⁵ ist, R³ H oder CH₃ ist und R⁴ und R⁵ unabhängig voneinander jeweils H oder C₁-C₃-Alkyl sind,
Y = -PO(OH)₂, -O-PO(OH)₂, -SO₃H ist,
n = 1 oder 2 ist,
m = 1 oder 2 ist.

4. Dentalwerkstoff nach Anspruch 3, wobei
R¹, R² = unabhängig voneinander jeweils ein linearer aliphatischer C₂-C₁₀-Rest sind, der durch 1 oder 2 -O-unterbrochen sein kann, wobei R¹ und R² vorzugsweise gleich sind,
X = CH₂=CR³-CO-Z-, wobei Z O oder NR⁵ ist, oder R⁴O-CO-C(=CH₂)-CH₂-Z- ist, wobei Z = O ist, R³ H oder CH₃ ist und R⁴ Methyl oder Ethyl ist und R⁵ H, Methyl oder Ethyl ist,
Y = -PO(OH)₂, -O-PO(OH)₂, -SO₃H ist,
n, m = jeweils 1 sind.

5. Dentalwerkstoff nach Anspruch 1 oder 2, wobei
R¹ = ein aliphatischer C₂-C₁₂-Rest ist, der durch -O- oder -CO-O- unterbrochen sein kann,
R² = ein aliphatischer C₁-C₁₀-Rest ist, der durch -O- oder -CO-O- unterbrochen sein kann,
X, Y = unabhängig voneinander jeweils CH₂=CR³-CO-Z-oder R⁴O-CO-C(=CH₂)-CH₂-Z- sind, wobei Z O oder NR⁵ ist, R³ H oder CH₃ ist und R⁴ und R⁵ unabhängig voneinander jeweils H oder C₁-C₃-Alkyl sind, wobei X und Y vorzugsweise gleich sind,
n = 1 oder 2 ist,
m = 1 oder 2 ist.

6. Dentalwerkstoff nach Anspruch 5, wobei
R¹, R² = unabhängig voneinander jeweils ein linearer aliphatischer C₂-C₁₀-Rest sind, der durch 1 oder 2 -O-unterbrochen sein kann, wobei R¹ und R² vorzugsweise gleich sind,
X, Y = unabhängig voneinander jeweils CH₂=CR³-CO-Z-, wobei Z O oder NR⁵ ist, oder R⁴O-CO-C(=CH₂)-CH₂-Z-sind, wobei Z = O ist, R³ H oder CH₃ ist und R⁴ Methyl oder Ethyl ist und R⁵ H, Methyl oder Ethyl ist, wobei X und Y vorzugsweise gleich sind,
n, m = jeweils 1 sind.

7. Dentalwerkstoff nach einem der Ansprüche 1 bis 6, der zusätzlich mindestens einen Initiator für die radikalische Polymerisation enthält.

8. Dentalwerkstoff nach einem der Ansprüche 1 bis 7, der zusätzlich mindestes ein weiteres radikalisch polymerisierbares Monomer enthält.

9. Dentalwerkstoff nach Anspruch 8, der als weiteres Monomer ein oder mehrere mono- oder polyfunktionelle (Meth)acrylsäurederivate und/oder (Meth)acrylamidderivate enthält.

10. Dentalwerkstoff nach einem der Ansprüche 1 bis 9, der zusätzlich mindestens ein Lösungsmittel enthält, vorzugsweise Wasser oder eine Mischung aus Wasser und einem polaren organischen Lösungsmittel.

11. Dentalwerkstoff nach einem der Ansprüche 7 bis 10, der
a) 0,1 bis 50 Gel.-% Harnstoffderivat der allgemeinen Formel I,
b) 0,01 bis 10 Gew.-% Initiator,
c) 0 bis 80 Gew.-% weiteres Monomer,
d) 0 bis 80 Gew.-% Füllstoff,
e) ggf. 0 bis 10 Gew.-%, bevorzugt 0,1 bis 3 Gew.-% Additiv,
e) 0 bis 70 Gew.-% Lösungsmittel enthält.

12. Dentalwerkstoff nach Anspruch 11 zur Verwendung als Adhäsiv, der 0 bis 20 Gew.-% Füllstoff enthält.

13. Dentalwerkstoff nach Anspruch 11 zur Verwendung als Zement, Füllungsmaterial oder Beschichtungsmaterial, der 20 bis 80 Gew.-% Füllstoff enthält.

14. Dentalwerkstoff nach einem der Ansprüche 1 bis 13 zur intraoralen Verwendung.

15. Verwendung eines Dentalwerkstoffs nach einem der Ansprüche 1 bis 13 zur extraoralen Anfertigung oder Reparatur von Dentalrestaurationen.

## Claims

1. Dental material, **characterised in that** it comprises a urea derivative according to the general Formula I, 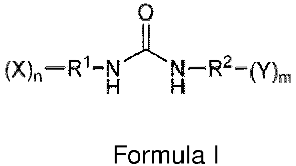 in which
R¹, R² = independently of one another are each an aliphatic C₁-C₁₅ group that may be interrupted by -O-, -S-, -CO-O-,
X = a radically polymerisable group,
Y = a radically polymerisable group or an acidic group,
n, m = independently of one other are 1, 2 or 3.

2. Dental material according to claim 1, **characterised in that**
X = CH₂=CR³-CO-Z- or R⁴O-CO-C(=CH₂)-CH₂-Z-, wherein Z is O or NR⁵ or is absent, R³ is H or CH₃ and R⁴ and R⁵ independently of one another are each H or C₁-C₇ alkyl, and/or
Y = CH₂=CR³'-CO-Z'- or R⁴'O-CO-C(=CH₂)-CH₂-Z'-, wherein Z' is O or NR⁵' or is absent, R³' is H or CH₃ and R⁴' and R⁵' independently of one another are each H or C₁-C₇ alkyl, or is -PO(OH)₂, -O-PO(OH)₂, -SO₃H.

3. Dental material according to claim 1 or 2, **characterised in that**
R¹ = an aliphatic C₂-C₁₂ group that may be interrupted by -O- or -CO-O-,
R² = an aliphatic C₁-C₁₀ group that may be interrupted by -O- or -CO-O-,
X = CH₂=CR³-CO-Z- or R⁴O-CO-C(=CH₂)-CH₂-Z-, wherein Z is O or NR⁵, R³ is H or CH₃ and R⁴ and R⁵ independently of one another are each H or C₁-C₇ alkyl,
Y = -PO(OH)₂, -O-PO(OH)₂, -SO₃H,
n = 1 or 2,
m = 1 or 2.

4. Dental material according to claim 3, **characterised in that**
R¹, R₂ = independently of one another are each a linear aliphatic C₂-C₁₀ group that may be interrupted by 1 or 2 -O-, wherein R¹ and R² are preferably identical,
X = CH₂=CR³-CO-Z-, wherein Z is O or NR⁵, or is R⁴O-CO-C(=CH₂)-CH₂-Z-, wherein Z = O, R³ is H or CH₃ and R⁴ is methyl or ethyl and R⁵ is H, methyl or ethyl,
Y = -PO(OH)₂, -O-PO(OH)₂, -SO₃H,
n, m are each 1.

5. Dental material according to claim 1 or 2, **characterised in that**
R¹ = an aliphatic C₂-C₁₂ group that may be interrupted by -O- or -CO-O-,
R² = an aliphatic C₁-C₁₀ group that may be interrupted by -O- or -CO-O-,
X, Y = each independently of one another are CH₂=CR³-CO-Z- or R⁴O-CO-C(=CH₂)-CH₂-Z-, wherein Z is O or NR⁵, R³ is H or CH₃ and R⁴ and R⁵ independently of one another are each H or C₁-C₃ alkyl, wherein X and Y are preferably identical,
n = 1 or 2,
m = 1 or 2.

6. Dental material according to claim 5, **characterised in that**
R¹, R² = independently of one another are each a linear aliphatic C₂-C₁₀ group that may be interrupted by 1 or 2 -O-, wherein R¹ and R² are preferably identical,
X, Y = each independently of one another are CH₂=CR³-CO-Z-, wherein Z is O or NR⁵, or R⁴O-CO-C(=CH₂)-CH₂-Z-, wherein Z is O, R³ is H or CH₃ and R⁴ is methyl or ethyl and R⁵ is H methyl or ethyl, wherein X and Y are preferably identical,
n, m are each 1.

7. Dental material according to one of claims 1 to 6, **characterised in that** it additionally comprises an initiator for the radical polymerisation.

8. Dental material according to one of claims 1 to 7, **characterised in that** it additionally comprises at least one other radically polymerisable monomer.

9. Dental material according to claim 8, **characterised in that** it comprises one or more mono- or polyfunctional (meth)acrylic acid derivatives and/or (meth)acrylamide derivatives as the one other monomer.

10. Dental material according to one of claims 1 to 9, **characterised in that** it further comprises at least one solvent, preferably water or a mixture of water and a polar organic solvent.

11. Dental material according to one of claims 7 to 10, **characterised in that** it comprises
a) 0.1 to 50 wt % urea derivative of the general Formula I,
b) 0.01 to 10 wt % initiator,
c) 0 to 80 wt % of another monomer,
d) 0 to 80 wt % filler,
e) optionally 0 to 10 wt %, preferably 0.1 to 3 wt % additive,
e) 0 to 70 wt % solvent.

12. Dental material according to claim 11 for use as an adhesive, which comprises 0 to 20 wt % filler.

13. Dental material according to claim 11 for use as a cement, filling material or coating material, which comprises 20 to 80 wt % filler.

14. Dental material according to one of claims 1 to 13 for intraoral use.

15. Use of a dental material according to one of claims 1 to 13 for the extraoral preparation or repair of dental restaurations.

## Revendications

1. Matériau dentaire, **caractérisé en ce qu'**il contient un dérivé d'urée, de formule générale I : 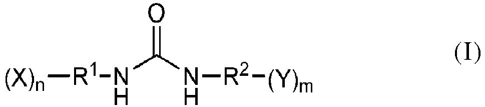 dans laquelle
- les symboles R¹ et R² représentent chacun, indépendamment l'un de l'autre, un groupe aliphatique en C₁-C₁₅, qui peut être interrompu par un chaînon -O-, -S- ou -CO-O-,
- X représente un groupe polymérisable par voie radicalaire,
- Y représente un groupe polymérisable par voie radicalaire ou un groupe acide,
- et les indices n et m valent chacun, indépendamment l'un de l'autre, 1, 2 ou 3.

2. Matériau dentaire conforme à la revendication 1, dans lequel
- X représente un groupe de formule
CH₂=CR³-CO-Z- ou R⁴O-CO-C(=CH₂)-CH₂-Z-,
où Z représente un chaînon -O- ou -NR⁵- ou ne représente rien, R³ représente un atome d'hydrogène ou un groupe méthyle, et R⁴ et R⁵ représentent chacun, indépendamment l'un de l'autre, un atome d'hydrogène ou un groupe alkyle en C₁-C₇,
- et/ou Y représente soit un groupe de formule
CH₂=CR³'-CO-Z'- ou R^{4'}O-CO-C(=CH₂)-CH₂-Z'-,
où Z' représente un chaînon -O- ou -NR⁵'- ou ne représente rien, R³' représente un atome d'hydrogène ou un groupe méthyle, et R⁴' et R⁵' représentent chacun, indépendamment l'un de l'autre, un atome d'hydrogène ou un groupe alkyle en C₁-C₇,
soit un groupe de formule -PO(OH)₂, -O-PO(OH)₂ ou -SO₃H.

3. Matériau dentaire conforme à la revendication 1 ou 2, dans lequel
- R¹ représente un groupe aliphatique en C₂-C₁₂, qui peut être interrompu par un chaînon -O- ou -CO-O-,
- R² représente un groupe aliphatique en C₁-C₁₀, qui peut être interrompu par un chaînon -O- ou -CO-O-,
- X représente un groupe de formule
CH₂=CR³-CO-Z- ou R⁴O-CO-C(=CH₂)-CH₂-Z-,
où Z représente un chaînon -O- ou -NR⁵-, R³ représente un atome d'hydrogène ou un groupe méthyle, et R⁴ et R⁵ représentent chacun, indépendamment l'un de l'autre, un atome d'hydrogène ou un groupe alkyle en C₁-C₃,
- Y représente un groupe de formule -PO(OH)₂, -O-PO(OH)₂ ou -SO₃H,
- l'indice n vaut 1 ou 2,
- et l'indice m vaut 1 ou 2.

4. Matériau dentaire conforme à la revendication 3, dans lequel
- R¹ et R² représentent chacun, indépendamment l'un de l'autre, un groupe aliphatique linéaire en C₂-C₁₀, qui peut être interrompu par un ou deux chaînon(s) -O-, étant entendu que R¹ et R² représentent de préférence des groupes identiques,
- X représente un groupe de formule CH₂=CR³-CO-Z- dans laquelle Z représente un chaînon -O- ou -NR⁵-, ou un groupe de formule R⁴O-CO-C(=CH₂)-CH₂-Z- dans laquelle Z représente un chaînon -O-, et où R³ représente un atome d'hydrogène ou un groupe méthyle, R⁴ représente un groupe méthyle ou éthyle, et R⁵ représente un atome d'hydrogène ou un groupe méthyle ou éthyle,
- Y représente un groupe de formule -PO(OH)₂, -O-PO(OH)₂ ou -SO₃H,
- et les indices n et m valent chacun 1.

5. Matériau dentaire conforme à la revendication 1 ou 2, dans lequel
- R¹ représente un groupe aliphatique en C₂-C₁₂, qui peut être interrompu par un chaînon -O- ou -CO-O-,
- R² représente un groupe aliphatique en C₁-C₁₀, qui peut être interrompu par un chaînon -O- ou -CO-O-,
- X et Y représentent chacun, indépendamment l'un de l'autre, un groupe de formule
CH₂=CR³-CO-Z- ou R⁴O-CO-C(=CH₂)-CH₂-Z-,
où Z représente un chaînon -O- ou -NR⁵-, R³ représente un atome d'hydrogène ou un groupe méthyle, et R⁴ et R⁵ représentent chacun, indépendamment l'un de l'autre, un atome d'hydrogène ou un groupe alkyle en C₁-C₃, étant entendu que X et Y représentent de préférence des groupes identiques,
- Y représente un groupe de formule -PO(OH)₂, -O-PO(OH)₂ ou -SO₃H,
- l'indice n vaut 1 ou 2,
- et l'indice m vaut 1 ou 2.

6. Matériau dentaire conforme à la revendication 5, dans lequel
- R¹ et R² représentent chacun, indépendamment l'un de l'autre, un groupe aliphatique linéaire en C₂-C₁₀, qui peut être interrompu par un ou deux chaînon(s) -O-, étant entendu que R¹ et R² représentent de préférence des groupes identiques,
- X et Y représentent chacun, indépendamment l'un de l'autre, un groupe de formule CH₂=CR³-CO-Z- dans laquelle Z représente un chaînon -O- ou -NR⁵-, ou de formule R⁴O-CO-C(=CH₂)-CH₂-Z-dans laquelle Z représente un chaînon -O-, et où R³ représente un atome d'hydrogène ou un groupe méthyle, R⁴ représente un groupe méthyle ou éthyle, et R⁵ représente un atome d'hydrogène ou un groupe méthyle ou éthyle, étant entendu que X et Y représentent de préférence des groupes identiques,
- et les indices n et m valent chacun 1.

7. Matériau dentaire conforme à l'une des revendications 1 à 6, qui contient en outre au moins un amorceur pour polymérisation par voie radicalaire.

8. Matériau dentaire conforme à l'une des revendications 1 à 7, qui contient en outre au moins un autre monomère polymérisable par voie radicalaire.

9. Matériau dentaire conforme à la revendication 8, qui contient, en tant qu'autre monomère, un ou plusieurs dérivé(s) monofonctionnel(s) ou polyfonctionnel(s) d'acide acrylique ou méthacrylique et/ou d'acrylamide ou de méthacrylamide.

10. Matériau dentaire conforme à l'une des revendications 1 à 9, qui contient en outre au moins un solvant, de préférence de l'eau ou un mélange d'eau et d'un solvant organique polaire.

11. Matériau dentaire conforme à l'une des revendications 7 à 10, qui contient
a) de 0,1 à 50 % en poids d'un dérivé d'urée de formule générale (I),
b) de 0,01 à 10 % en poids d'un amorceur,
c) de 0 à 80 % en poids d'un autre monomère,
d) de 0 à 80 % en poids d'une charge,
e) en option, de 0 à 10 % en poids et de préférence de 0,1 à 3 % en poids d'un adjuvant,
f) et de 0 à 70 % en poids d'un solvant.

12. Matériau dentaire conforme à la revendication 11 pour utilisation comme adhésif, qui contient de 0 à 20 % en poids d'une charge.

13. Matériau dentaire conforme à la revendication 11 pour utilisation comme ciment, matériau de remplissage ou matériau de revêtement, qui contient de 20 à 80 % en poids d'une charge.

14. Matériau dentaire conforme à l'une des revendications 1 à 13, pour utilisation à l'intérieur de la bouche.

15. Utilisation d'un matériau dentaire conforme à l'une des revendications 1 à 13 pour la confection ou la réparation, à l'extérieur de la bouche, de pièces de restauration dentaire.
